(19) Europäisches Patentamt
European Patent Office
Office européen des brevets

(11) Veröffentlichungsnummer: **0 266 690 B1**

# (12) EUROPÄISCHE PATENTSCHRIFT

(45) Veröffentlichungstag der Patentschrift: **05.02.92**

(51) Int. Cl.5: **C07C 69/63**, C07C 69/635, C07C 69/708, C07C 67/307

(21) Anmeldenummer: **87115958.8**

(22) Anmeldetag: **30.10.87**

## (54) Verfahren zur Herstellung von 2,2-disubstituierten 3-Chlorpropionsäureestern.

(30) Priorität: **07.11.86 DE 3638009**

(43) Veröffentlichungstag der Anmeldung:
**11.05.88 Patentblatt 88/19**

(45) Bekanntmachung des Hinweises auf die Patenterteilung:
**05.02.92 Patentblatt 92/06**

(84) Benannte Vertragsstaaten:
**DE FR GB IT NL**

(56) Entgegenhaltungen:
**EP-A- 0 173 976**
**DE-A- 2 003 238**
**GB-A- 2 051 808**

**CHEMISCHES CENTRALBLATT, Band 75, 1904.II, Seiten 1106-1107, Berlin; A. FRANKE et al: "Ueber eine kondensierende Wirkung des Magnesiumäthyljodids"**

(73) Patentinhaber: **BASF Aktiengesellschaft**
**Carl-Bosch-Strasse 38**
**W-6700 Ludwigshafen(DE)**

(72) Erfinder: **Merger, Franz, Dr.**
**Max-Slevogt-Strasse 25**
**W-6710 Frankenthal(DE)**
Erfinder: **Hettinger, Peter, Dr.**
**Schloss-Strasse 3**
**W-6803 Edingen-Neckarhausen(DE)**

**EP 0 266 690 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von 2,2-disubstituierten 3-Chlorpropionsäureestern der Formel I

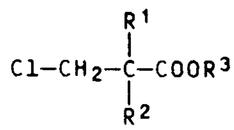

I,

in der die Reste $R^1$ und $R^2$ einen $C_1$- bis $C_6$-Alkylrest, einen $C_2$- bis $C_6$-Oxaalkyl-, Alkenyl-, Oxaalkenyrest, einen Aryl- oder einen $C_7$- bis $C_{12}$-Aralkylrest darstellen, wobei $R^1$-C-$R^2$ auch miteinander einen 5- bis 7-gliedrigen Ring bilden können und in der $R^3$, abgesehen von Aryl, die für $R^1$ und $R^2$ angegebene Bedeutung hat.

Gemäß dem Stand der Technik lassen sich 2,2-disubstituierte 3-Chlorpropionsäureester, die wertvolle Zwischenprodukte für die Herstellung von Isoxazolidin-3-onen darstellen, welche als Bausteine für Pflanzenschutzmittel verwendet werden, durch Veresterung von entsprechend substituierten 3-Chlorpropionsäuren oder -säurechloriden herstellen. Dieser Syntheseweg ist jedoch relativ aufwendig und wirtschaftlich wenig attraktiv, da die Ausgangsstoffe nicht leicht zugänglich sind. Durch die bekannte, schwierig lenkbare, von Korrosionsproblemen belastete Monochlorierung von Neocarbonsäuren sind z.B. Chlorpropionsäuren nur in engen Grenzen überhaupt zugänglich.

Der Erfindung lag daher die Aufgabe zugrunde, die Herstellung von 2,2-disubstituierten 3-Chlorpropionsäureestern I, ausgehend von billigen, leicht verfügbaren Ausgangsstoffen, in größerer Variationsbreite zu ermöglichen.

Demgemäß wurde ein Verfahren zur Herstellung von 2,2-disubstituierten 3-Chlorpropionsäureestern der Formel I

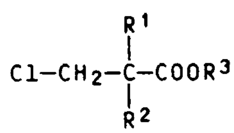

I,

in der die Reste $R^1$ und $R^2$ einen C- bis $C_6$-Alkylrest, einen $C_2$- bis $C_6$-Oxaalkyl-, Alkenyl-, Oxaalkenylrest, einen Aryl- oder einen $C_7$- bis $C_{12}$-Aralkylrest darstellen, wobei $R^1$ -C- $R^2$ auch miteinander einen 5- bis 7-gliedrigen Ring bilden können und in der $R^3$ abgesehen von Aryl, die für $R^1$ und $R^2$ angegebene Bedeutung hat, gefunden, welches dadurch gekennzeichnet ist, daß man 2,2-disubstituierte 3-Hydroxypropanale der Formel II

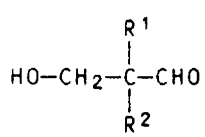

II

in das Esterdiol III

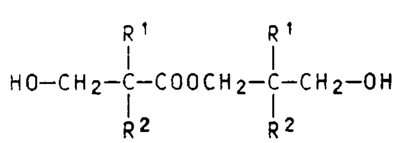

III

überführt, dieses in Gegenwart eines Umesterungskatalysators mit einem Alkohol $R^3OH$ zum 3-Hydroxyester IV umsetzt

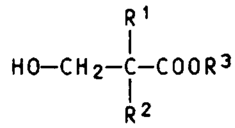

IV,

und den Hydroxyester IV mit stöchiometrischen oder mehr als stöchiometrischen Mengen Thionylchlorid zum Chlorpropionsäureester I umsetzt.

Das Gesamtverfahren läßt sich durch folgende Reaktionsgleichungen wiedergeben:

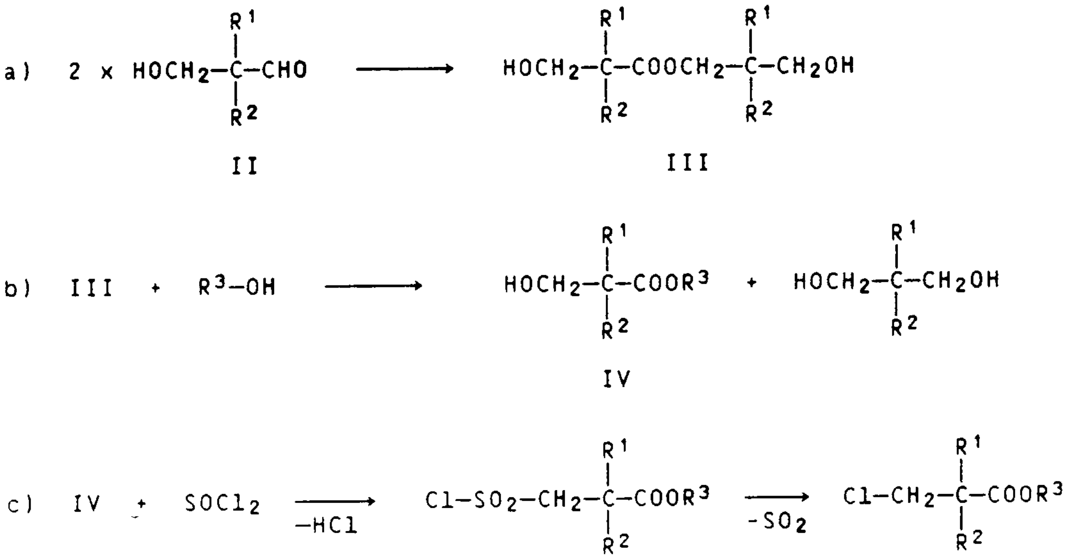


a) II III

b) IV

c) I

Nach diesem Verfahren lassen sich die Endstoffe I in guten Ausbeuten (bis über 70 %, bezogen auf den Ausgangsstoff II) herstellen. Der Erfolg des Verfahrens ist überraschend, da sowohl in Stufe b) als auch Stufe c) Nebenreaktionen, bedingt durch die Struktur der Ester III bzw. IV zu erwarten waren. So war im Fall der Umesterungsreaktion b) anzunehmen, daß die Alkoholgruppen in den Esterdiolen III in Konkurrenz zum eingesetzten Alkohol $R^3OH$ treten würden, was zu Oligo- und Polyesterbildung geführt hätte. Gemäß dem Stand der Technik hätte man angenommen, daß der Austausch der Hydroxygruppe gegen Chlor in Stufe c), aufgrund der sterisch gehinderten Struktur von IV allenfalls mit unbefriedigender Ausbeute verläuft. So geht z.B. aus J. Chem. Soc. 3640 bis 3641 (1954) hervor, daß die thermische Zersetzung des Neopentylchlorsulfinates in Gegenwart von Chinolinhydrochlorid bei 115° C zu Neopentylchlorid nur in einer Ausbeute von 47 % abläuft.

Die für das erfindungsgemäße Verfahren benötigten Ausgangsstoffe II sind leicht zugänglich, z.B. durch Aldolreaktion von 2,2-disubstituierten Acetaldehyden mit Formaldehyd (siehe Houben-Weyl, Methoden der organischen Chemie, Bd. 7.1, S. 89ff (1954)) oder gemäß den DE-OS 17 93 512 und 19 57 301 sowie durch Umsetzung von 2-Alkylacroleinen mit Alkoholen und Formaldehyd gemäß der DE-OS 33 21 517. Als Reste $R^1$ und $R^2$ kommen $C_1$- bis $C_6$-Alkylreste, $C_2$- bis $C_6$-Oxaalkyl-, Alkenyl-, Oxaalkenylreste, Aryl- und $C_7$- bis $C_{12}$-Aralkylreste in Betracht. Beispielsweise seien der Methyl-, Ethyl-, Propyl-, iso-Propyl-, Butyl-, Pentyl-, Hexyl-, Methoxmethyl-, Ethoxmethyl-, Methoxyethyl- oder Isopropoxyethylrest sowie der Allyl-, Phenyl-, Phenylethyl- oder Benzylrest genannt. Der Reste $R^1$ und $R^2$ können auch miteinander einen 5- bis 7-gliedrigen Ring bilden, der ein oder mehrere, insbesondere 1 oder 2 Sauerstoffatome enthalten kann, z.B. können $R^1$ und $R^2$ zusammen mit dem C-Atom, an das sie gebunden sind, einen Cyclopentyl-, Cyclohexen-3-yl-, Cyclohexyl-, Tetrahydrofuryl-(2), Tetrahydropyranyl-(2)- oder einen 1,4-Dioxacyclohexylrest bilden. Vorzugsweise steht $R^1$ für einen $C_1$- bis $C_6$-Alkylrest und $R^2$ für einen $C_1$- bis $C_6$-Alkyl-, Oxaalkyl-, Phenyl- oder Benzylrest oder die Reste $R^1$ und $R^2$ bilden zusammen einen $C_5$- bis $C_7$-Cycloalkylrest, der 1 oder 2 Sauerstoffatome enthalten kann.

Die Herstellung der Ester III durch Disproportionierung der Ausgangsstoffe II nach Tischtschenko kann in an sich bekannter Weise erfolgen, z.B. durch Erhitzen der 2,2-disubstituierten 3-Hydroxypropanale in Abwesenheit oder in Gegenwart von Katalysatoren, vorzugsweise nach den in den DE-OS 22 34 358, 25 00

311 und 34 32 577 beschriebenen Verfahren. In einer bevorzugten, wirtschaftich besonders vorteilhaften Ausführungsform des Verfahrens wird das Produkt III, gegebenenfalls nach Abtrennung von nicht umgesetztem Ausgangsstoff ohne Reinigung direkt weiterverarbeitet.

Stufe b) des Verfahrens kann in Gegenwart gängiger Umesterungskatalysatoren z. B. Alkoholaten durchgeführt werden. Besonders bevorzugte Katalysatoren sind Erdalkali- oder insbesondere Akalialkoholate, z. B. Magnesium-, Calcium-, Lithium-, Natrium- oder Kaliumalkoholate, wobei hinsichtlich der Alkoholkomponente kaum Beschränkungen bestehen. So kann man vorzugsweise $C_1$- bis $C_8$-Alkoholate und vorteilhaft die Alkoholate der eingesetzten Alkohole $R^3OH$ verwenden.

Als Alkohole $R^3OH$ wird vorzugsweise Methanol verwendet, wenn die Endprodukte zu Isoxazolidinonen weiterverarbeitet werden sollen. Ferner kann $R^3$ einen $C_1$- bis $C_6$-Alkylrest, einen $C_2$- bis $C_6$-Oxaalkyl-, Alkenyl- oder Oxaalkenylrest sowie einen C7- bis $C_{12}$-Aralkylrest bedeuten. Beispielsweise seien Methanol, Ethanol, Propanol, iso-Propanol, n-Butanol, iso-Butanol, tert. -Butanol, n-Pentanol, iso-Pentanol, n- oder iso-Hexanol, Methoxiethanol, Butoxiethanol, Allylalkohol oder Methallylalkohol genannt. Aralkylalkohole sind beispielsweise Benzylalkohol oder 2-Phenylethanol-(1).

Die Umesterung kann bei Temperaturen von -10 bis +50 °C, vorzugsweise 10 bis 40, insbesondere 20 bis 35 °C vorgenommen werden. Die Menge des eingesetzten Alkohols $R^3OH$ ist nicht prinzipiell kritisch und kann zwischen 1 und 20 mol, vorteilhaft 2 bis 15 mol, insbesondere 5 bis 12 mol pro Mol Esterdiol III betragen. An Base werden nur katalytische Mengen benötigt. Im Fall von Alkalialkoholaten kann man vorzugsweise 0,01 bis 0,1 mol je Mol Esterdiol III verwenden. Besonders beim Einsatz von $R^3OH$ im unteren Molverhältnis kann die Abtrennung von nicht umgesetztem Esterdiol und die Rückführung empfehlenswert sein. Die Isolierung der Reaktionsprodukte aus dem Umsetzungsgemisch kann vorteilhaft durch Destillation nach den dafür üblichen Techniken erfolgen.

Die so erhaltenen 2,2-disubstituierten 3-Hydroxyester IV werden durch Umsetzung mit stöchiometrischen oder mehr als stöchiometrischen Mengen Thionylchlorid in den Chlorpropionsäureester I übergeführt. Vorteilhaft kann man 1 bis 3 mol Thionylchlorid je Mol IV verwenden, höhere Mengen sind möglich, bringen aber keine Vorteile.

Die Umsetzung von IV mit Thionylchlorid wird vorzugsweise in 2 Temperaturintervallen vorgenommen werden, indem man zunächst bei -10 bis 60, vorzugsweise 0 bis 60, insbesondere 10 bis 50 °C im wesentlichen zum Chlorsulfinat umsetzt und dann die Temperatur bis auf 180 °C erhöht. Vor der thermischen Spaltung des intermediär gebildeten Chlorsulfinates wird man vorteilhaft gegebenenfalls im Reaktionsgemisch vorliegendes überschüssiges Thionylchlorid durch Destillation abtrennen.

Die Spaltung des Chlorsulfinates in $SO_2$ und Chlorpropionsäureester I kann in Gegenwart katalytischer Mengen an tertiären Stickstoffbasen wie Pyridin, Chinolin oder Dimethylanilin oder deren Salzen, z.B. den Hydrochloriden erfolgen. Im allgemeinen liegen die Temperaturen im Bereich von 120 bis 180 °C, insbesondere 120 bis 140 °C.

Das erfindungsgemäße Verfahren ermöglicht, ausgehend von leicht zugänglichen Ausgangsstoffen, eine wirtschaftliche Herstellung der als Vorprodukte von Pflanzenschutzmitteln dienenden 2,2-disubstituierten 3-Chlorpropionsäureester. Die in der Stufe b) des Verfahrens anfallenden substituierten 1,3-Propandiole stellen in der Regel zusätzliche Wertprodukte dar, beispielsweise läßt sich Neopentylglykol als Komponente für Polyester oder Polyurethane verwenden.

Beispiel 1

Herstellung von 2,2-Dimethyl-3-chlorpropionsäuremethylester

101 g (1,25 mol) 37 %ige wäßrige Formaldehydlösung und 99 g (1,37 mol) Isobutyraldehyd wurden bei 40 °C unter Stickstoff durch Rühren mit 4,2 g (0,03 mol) 40 %iger wäßriger Trimethylaminlösung gemischt. Die Temperatur des Gemisches stieg im Verlauf von 15 bis 20 Minuten auf 93 bis 94 °C. Man rührte das Gemisch noch 10 Minuten bei dieser Temperatur und destillierte dann unter vermindertem Druck das Trimethylamin, den überschüssigen Isobutyraldehyd sowie 58 g Wasser ab. Anschließend wurde die verbleibende Lösung von Hydroxypivalinaldehyd auf 60 °C abgekühlt und unter kräftigem Rühren mit 1,9 g (0,025 mol) fein pulverisiertem Calciumhydroxid versetzt. Nach Abklingen der Wärmeentwicklung und kurzem Nachrühren wurde bei 70 °C 2,5 g (0,054 mol) Ameisensäure (100 %) zugesetzt und die Mischung bei 70 °C 10 Minuten lang gerührt. Anschließend wurde die wäßrige Phase abgetrennt und die organische Phase fraktionierend destilliert. Man isolierte 110 g (0,54 mol) 2,2-Dimethyl-1,3-propandiolhydroxipivalinsäure-monoester, entsprechend 86,5 % der Theorie, bezogen auf den eingesetzten Formaldehyd.

136 g (0.66 mol) 2,2-Dimethyl-1,3-propandiolhydroxipivalinsäure-monoester wurden in 149 ml Methanol

gelöst, mit 1,3 g (0,02 mol) Natriummethanolat versetzt und bei Raumtemperatur 16 h lang gerührt.

Anschließend neutralisierte man die Reaktionsmischung mit Essigsäure und arbeitete das Gemisch durch fraktionierende Destillation auf. Man erhielt nach Abtrennen des Methanols 78 g (0,59 mol) Hydroxipivalinsäuremethylester vom $Sdp_{175}$ = 135 °C sowie 58 g (0,56 mol) 2,2-Dimethyl-1,3-propandiol, was einer Ausbeute von 89 bzw. 84,5 %, bezogen auf eingesetztes Esterdiol entspricht.

Zu 238 g (2 mol) Thionylchlorid wurden 132 g (1 mol) Hydroxipivalinsäuremethylester unter Kühlung so zugegeben, daß die Temperatur der Reaktionsmischung 20 °C nicht überstieg. Nach beendeter Zugabe rührte man das Gemisch 1 h bei 50 °C. Anschließend wurde überschüssiges Thionylchlorid bei Normaldruck abdestilliert, der Rückstand mit 0,2 g (0,0025 mol) Pyridin versetzt und auf 135 °C erhitzt.

Nach beendeter $SO_2$-Entwicklung isolierte man durch Destillation 138 g (0,92 mol) 2,2-Dimethyl-3-chlorpropionsäuremethylester ($Kp_5$ = 82 °C) in 92 % Ausbeute, bezogen auf Hydroxipivalinsäuremethylester.

Beispiel 2

Zu einem auf 50 °C erwärmten Gemisch aus 975 g (12 mol) einer 37 %igen Formaldehydlösung und 865 g (12 mol) Isobutyraldehyd ließ man unter kräftigem Rühren und Rückflußkühlung 120 g (1,18 mol) Triethylamin zulaufen und die Temperatur im Verlauf von 10 Minuten auf 92 bis 94 °C ansteigen. Dann wurden Triethylamin und Wasser innerhalb 15 bis 20 Minuten bei 150 bis 30 mbar und einer maximalen Sumpftemperatur von 70 bis 75 °C abdestilliert. Anschließend heizte man das verbleibende Gemisch bei Normaldruck innerhalb 15 Minuten auf 160 °C, wobei im wesentlichen noch Reste von Wasser abdestillierten, und rührte es bei dieser Temperatur 160 Minuten weiter. Durch Destillation trennte man aus dem Gemisch 180 g einer Fraktion vom Siedebereich 80 bis 95 °C bei 8 bis 10 mbar ab, die 119 g (1,17 mol) Hydroxipivalinaldehyd, entsprechend 9,75 %, bezogen auf die Ausgangsstoffe, enthielt. Es verblieben 305 g Rückstand, der nach gaschromatographischer Analyse aus 96 % (869 g; 4,26 mol) 2,2-Dimethylpropandiol-(1,3)-hydroxipivalinsäuremonoester, entsprechend 71 % Ausbeute, bezogen auf die Ausgangsstoffe (Gesamtselektivität 80,75 %), bestand. Den Rückstand nahm man in 1100 ml Methanol auf, gab 15 g (0,28 mol) Natriummethanolat zu, und rührte das Gemisch 15 Stunden bei Raumtemperatur. Nach Neutralisation mit Essigsäure und fraktionierender Destillation erhielt man 496,5 g (3,76 mol) Hydroxipivalinsäuremethylester, entsprechend 88,34 % Ausbeute bezogen auf das Esterdiol III, sowie 375,4 g (3,68 mol) 2,2-Dimethyl-1,3-propandiol, entsprechend 86,4 % Ausbeute.

Den Hydroxipivalinsäuremethylester gab man bei einer Temperatur unter 20 °C zu 893 g (7,5 mol) Thionylchlorid und rührte das Gemisch 1 Stunde bei 50 °C. Dann destillierte man das überschüssige Thionylchlorid bei Normaldruck ab, setzte dem Rückstand 1 g (0,0125 mol) Pyridin zu und erhitzte auf 135 °C.

Nach beendeter $SO_2$-Entwicklung isolierte man durch Destillation 524,8 g (3,48 mol) 2,2-Dimethyl-3-chlor-propionsäuremethylester (Sdp = 82 °C/5 mbar), entsprechend 92,7 % Ausbeute, bezogen auf Hydroxipivalinsäuremethylester.

Beispiel 3

232 g (2 mol) 2-Methyl-2-ethyl-3-hydroxipropanal und 12 g Wasser wurden auf 50 °C erwärmt und mit 4,5 g (0,06 mol) Calciumhydroxid versetzt. Nach Abklingen der Wärmeentwicklung rührte man bei 70 °C 30 min nach, neutralisierte mit Ameisensäure und entfernte das Salz durch wäßrige Extraktion. Anschließend destillierte man aus dem Produktgemisch bis zum Siedepunkt des 2-Methyl-2-ethyl-1,3-propandiol-2-methyl-2-ethyl-3-hydroxipropionsäureester (Sdp. = 140 bis 142 °C) 42 g Vorlauf ab.

Den Rückstand nahm man in 240 ml Methanol auf, fügte 2,5 g (0,046 mol) Natriummethylat hinzu und rührte das Gemisch 16 h bei Raumtemperatur. Nach Neutralisation mit Essigsäure erhielt man durch fraktionierende Destillation 108,9 g (0,74 mol) 2-Methyl-2-ethyl-3-hydroxipropionsäuremethylester vom Sdp. 100 °C/30 mbar, entsprechend 74,6 % Ausbeute, bezogen auf eingesetztes 2-Methyl-2-ethyl-3-hydroxipropanal sowie 89,7 g 2-Methyl-2-ethyl-1,3-propandiol, entsprechend 76 % Ausbeute.

Den 2-Methyl-2-ethyl-3-hydroxipropionsäuremethylester gab man bei einer Temperatur unterhalb von 20 °C zu 178,6 g (1.5 mol) Thionylchlorid und rührte das Gemisch noch 30 min bei 50 °C. Anschließend destillierte man das überschüssige Thionylchlorid bei 100 mbar ab, setzte zum Rückstand 0,5 g (0,006 mol) Pyridin zu und erhitzte 45 min auf 130 bis 135 °C. Durch fraktionierende Destillation isolierte man 114,4 g 2-Methyl-2-ethyl-3-chlorpropionsäuremethylester vom Sdp 101 bis 104 °C/8 mbar, entsprechend 93,2 % Ausbeute, bezogen auf 2-Methyl-2-ethyl-3-hydroxipropionsäuremethylester. Die Gesamtausbeute betrug 69,5 %, bezogen auf 2-Methyl-2-ethyl-3-hydroxipropanal.

Beispiel 4

430 g (3 mol) 1-Hydroximethyl-cyclohexylaldehyd wurden entsprechend Beispiel 3 bei -95 °C mit 7 g (0,095 mol) pulverisiertem Calciumhydroxid umgesetzt. Dann setzte man 100 g Wasser und 10 g Ameisensäure zu und entfernte das Salz durch wäßrige Extraktion. Anschließend destillierte man aus dem Produktgemisch bis zum Siedepunkt des 1,1-Bis-hydroximethylcyclohexanmono-(1'-hydroximethyl)-cyclohexancarbonsäureesters ($Sdp_3$ = 196 bis 198 °C/3 mbar) 69 g Vorlauf ab.

Den Rückstand nahm man in 350 ml Methanol auf, gab 3,75 g (0,069 mol) Natriummethanolat zu und rührte das Gemisch 15 Stunden bei Raumtemperatur. Nach Neutralisation mit Essigsäure erhielt man durch fraktionierende Destillation 179,8 g (1,046 mol) 1-Hydroximethylcyclohexancarbonsäuremethylester vom Sdp. = 105 bis 115 °C/1-2 mbar, entsprechend 69,7 % Ausbeute, bezogen auf eingesetzten 1-Hydroximethylcyclohexylaldehyd.

Den Hydroxiester setzte man wie in Beispiel 3 beschrieben mit 238 g (2 mol) Thionylchlorid um und erhitzte in Gegenwart von 1 g (0,0125 mol) Pyridin auf 135 °C. Nach beendeter $SO_2$-Entwicklung isolierte man durch Destillation 187,3 g (0,98 mol) 1-Chlormethylcyclohexancarbonsäuremethylester vom Sdp. = 95 bis 110 °C/12 mbar, entsprechend 65,3 % Ausbeute, bezogen auf umgesetzten 1-Hydroximethyl-cyclohexylaldehyd.

Beispiel 5

Zu einer auf 60 °C erwärmten Mischung von 750 g (5,68 mol) 2-Methoximethyl-2-methyl-3-hydroxipropanal wurden 37,5 g (0,51 mol) Calciumhydroxid gegeben. Dann steigerte man die Temperatur auf 80 °C und rührte 2 Stunden bei dieser Temperatur weiter. Anschließend wurde mit Ameisensäure neutralisiert und das Salz anschließend durch Filtration entfernt. Anschließend destillierte man aus dem Produktgemisch bis zum Siedepunkt des 2-Methoximethyl-2-methyl-1,3-propandiol-mono-2'-methoximethyl-2'-methyl-3-hydroxipropionats (Sdp. = 155 °C/2 mbar) 148 g Vorlauf ab.

Den Rückstand nahm man in 900 ml Methanol auf, gab 3,76 g (0,07 mol) Natriummethanolat zu und rührte 16 Stunden bei Raumtemperatur. Neutralisation mit Essigsäure und anschließende Destillation ergaben 312,8 g (1,93 mol) 2-Methoximethyl-2-methyl-3-hydroxipropionsäuremethylester vom Sdp. = 85 bis 95 °C/8 mbar, entsprechend 68 % Ausbeute, bezogen auf eingesetztes 2-Methoximethyl-2-methyl-3-hydroxipropanal und 249,7 g (1,86 mol) 2-Methoximethyl-2-methyl-1,3-propandiol vom Sdp. 108 bis 109 °C/8 mbar, entsprechend 66 % Ausbeute, bezogen auf eingesetztes 2-Methoximethyl-2-methyl-3-hydroxipropanal.

Den 2-Methoximethyl-2-methyl-3-hydroxipropionsäuremethylester gab man bei 15 bis 20 °C zu 438 g (3,68 mol) Thionylchlorid, steigerte nach erfolgter Zugabe die Temperatur auf 60 °C und rührte, bis die Gasentwicklung beendet war. Überschüssiges Thionylchlorid wurde abdestilliert, der Rückstand mit 0,2 g (0,0025 mol) Pyridin versetzt und auf 135 °C erhitzt. Nach beendeter $SO_2$-Entwicklung erhielt man durch Destillation 317,0 g (1,76 mol) 2-Methoximethyl-2-methyl-3-chlor-propionsäuremethylester vom Siedepunkt 94 °C/35 mbar, entsprechend 91 % Ausbeute, bezogen auf 2-Methoximethyl-2-methyl-3-hydroxipropionsäuremethylester.

**Patentansprüche**

1. Verfahren zur Herstellung von 2,2-disubstituierten 3-Chlorpropionsäureestern der Formel I

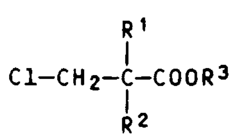

I,

in der die Reste $R^1$ und $R^2$ einen $C_1$- bis $C_6$-Alkylrest, einen $C_2$- bis $C_6$-Oxaalkyl-, Alkenyl-, Oxoalkenylrest, einen Aryl- oder einen $C_7$- bis $C_{12}$-Aralkylrest darstellen, wobei $R^1$-C-$R^2$ auch miteinander einen 5- bis 7-gliedrigen Ring bilden können und in der $R^3$, abgesehen von Aryl, die für $R^1$ und $R^2$ angegebene Bedeutung hat, dadurch gekennzeichnet, daß man 2,2-disubstituierte 3-Hydroxypropanale der Formel II

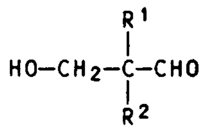 II

in das Esterdiol III

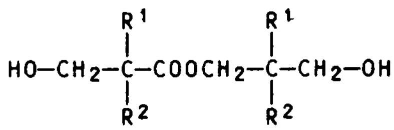 III

überführt, dieses in Gegenwart eines Umesterungskatalysators mit einem Alkohol $R^3OH$ zum 3-Hydroxyester IV umsetzt

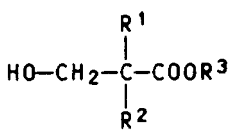 IV,

und den Hydroxyester IV mit stöchiometrischen oder mehr als stöchiometrischen Mengen Thionylchlorid zum Chlorpropionsäureester I umsetzt.

2. Verfahren nach Anspruch 1, dadurch gekennzeichnet, daß $R^3$ einen Methyl- oder Ethylrest bedeutet.

3. Verfahren nach den Ansprüchen 1 und 2, dadurch gekennzeichnet, daß man als Umesterungskatalysatoren Alkali- oder Erdalkalialkoholate verwendet.

4. Verfahren nach den Ansprüchen 1 bis 3, dadurch gekennzeichnet, daß man die Alkoholate in einer Menge von 0,01 bis 0,1 mol pro Mol Esterdiol III verwendet.

5. Verfahren nach den Ansprüchen 1 bis 4, dadurch gekennzeichnet, daß man 1 bis 20 mol Alkohol $R^3OH$ pro Mol des Esterdiols III verwendet.

6. Verfahren nach den Ansprüchen 1 bis 5, dadurch gekennzeichnet, daß man 1 bis 3 mol Thionylchlorid je Mol 3-Hydroxyester IV verwendet.

7. Verfahren nach den Ansprüchen 1 bis 6, dadurch gekennzeichnet, daß man die Umsetzung mit Thionylchlorid zunächst bei Temperaturen von -10 bis +60 °C und dann bei höherer Temperatur bis zu 180 °C durchführt.

8. Verfahren nach Anspruch 7, dadurch gekennzeichnet, daß man die Umsetzung zunächst bei 10 bis 50 °C und dann bei 120 bis 140 °C durchführt.

9. Verfahren nach Anspruch 7, dadurch gekennzeichnet, daß man die Umsetzung im oberen Temperaturbereich in Gegenwart einer tertiären Stickstoffbase durchführt.

## Claims

1. A process for the preparation of 2,2-disubstituted 3-chloropropionic esters of the formula I

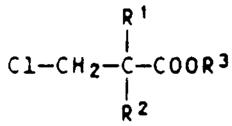

I

where $R^1$ and $R^2$ are each $C_1$-$C_6$-alkyl, $C_2$-$C_6$-oxaalkyl, alkenyl, oxoalkenyl, aryl or $C_7$-$C_{12}$-aralkyl, it also being possible for $R^1$-C-$R^2$ to form a 5- to 7-membered ring, and where $R^3$ has the meaning of $R^1$ and $R^2$ except for aryl, which comprises converting 2,2-disubstituted 3-hydroxypropanals of the formula II

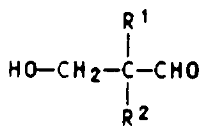

II

into the ester diol III

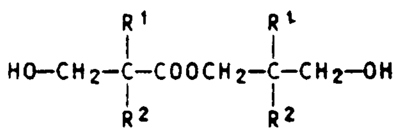

III

reacting the latter with an alcohol $R^3OH$ in the presence of a transesterification catalyst to give the 3-hydroxy ester IV

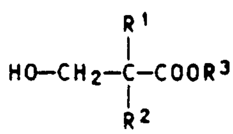

IV

and reacting the hydroxy ester IV with stoichiometric or more than stoichiometric amounts of thionyl chloride to give the chloropropionic ester I.

**2.** A process as claimed in claim 1, wherein $R^3$ is methyl or ethyl.

**3.** A process as claimed in claims 1 and 2, wherein alkali metal or alkaline earth metal alcoholates are used as transesterification catalysts.

**4.** A process as claimed in claims 1 to 3, wherein the alcoholates are used in an amount of from 0.01 to 0.1 mol per mole of ester diol III.

**5.** A process as claimed in claims 1 to 4, wherein from 1 to 20 mol of alcohol $R^3OH$ are used per mole of ester diol III.

**6.** A process as claimed in claims 1 to 5, wherein from 1 to 3 mol of thionyl chloride are used per mole of 3-hydroxy ester IV.

**7.** A process as claimed in claims 1 to 6, wherein the reaction with thionyl chloride is carried out initially at from -10 to +60 °C and then at up to 180 °C.

**8.** A process as claimed in claim 7, wherein the reaction is carried out initially at from 10 to 50 °C and then at from 120 to 140 °C.

9. A process as claimed in claim 7, wherein the reaction is carried out in the upper temperature range in the presence of a tertiary nitrogen base.

**Revendications**

1. Procédé de préparation d'esters d'acide 3-chloropropionique 2,2-disubstitué de formule I

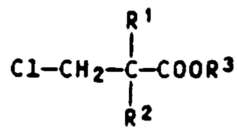

I,

dans laquelle les restes $R^1$ et $R^2$ représentent un reste alkyle en $C_1$-$C_6$, un reste oxaalkyle, alcényle ou oxoalcényle en $C_2$-$C_6$, un reste aryle ou un reste aralkyle en $C_7$-$C_{12}$, $R^1$-C-$R^2$ pouvant aussi former ensemble un noyau à 5-7 chaînons et dans laquelle $R^3$ a la signification donnée pour $R^1$ et $R^2$ à l'exception de aryle, caractérisé en ce qu'on transforme les 3-hydroxypropanals 2,2-disubstitués de formule II

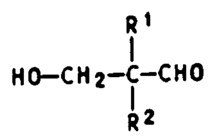

II

en l'esterdiol III

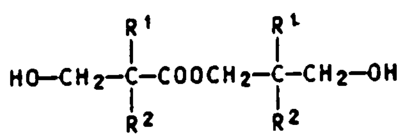

III ,

on fait réagir celui-ci avec un alcool $R^3OH$ en présence d'un catalyseur de transestérification pour obtenir le 3-hydroxyester IV

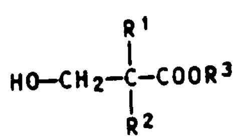

IV,

et on fait réagir l'hydroxyester IV avec des quantités stoechiométriques ou plus que stoechiométriques de chlorure de thionyle pour obtenir l'ester d'acide chloropropionique I.

2. Procédé selon la revendication 1, caractérisé en ce que $R^3$ représente un reste méthyle ou éthyle.

3. Procédé selon les revendications 1 et 2, caractérisé en ce qu'on utilise des alcoolates de métaux alcalins ou alcalino-terreux comme catalyseurs de transestérification.

4. Procédé selon les revendications 1 à 3, caractérise en ce qu'on utilise les alcoolates en une quantité de 0,01 à 0,1 mole par mole d'esterdiol III.

5. Procédé selon les revendications 1 à 4, caractérisé en ce qu'on utilise de 1 à 20 moles d'alcool $R^3OH$ par mole d'esterdiol III.

6. Procédé selon les revendications 1 à 5, caractérisé en ce qu'on utilise de 1 à 3 moles de chlorure de thionyle par mole de 3-hydroxyester IV.

7. Procédé selon les revendications 1 à 6, caractérisé en ce qu'on effectue la réaction avec le chlorure de thionyle d'abord à des températures de -10 à +60°C plus à des températures plus élevées allant jusqu'à 180°C.

8. Procédé selon la revendication 7, caractérisé en ce qu'on effectue la réaction d'abord à 10-50°C puis à 120-140°C.

9. Procédé selon la revendication 7, caractérisé en ce qu'on effectue la réaction dans la gamme de température supérieure en présence d'une base azotée tertiaire.